(19) Europäisches Patentamt

**European Patent Office**

**Office européen des brevets**

(11) Veröffentlichungsnummer: **0 210 552 B1**

(12) **EUROPÄISCHE PATENTSCHRIFT**

(45) Veröffentlichungstag der Patentschrift: **20.01.93**

(51) Int. Cl.⁵: **A61K 39/205**

(21) Anmeldenummer: **86109862.2**

(22) Anmeldetag: **18.07.86**

(54) **Tollwut-Lebendimpfstoff.**

(30) Priorität: **26.07.85 DE 3526809**

(43) Veröffentlichungstag der Anmeldung:
**04.02.87 Patentblatt 87/06**

(45) Bekanntmachung des Hinweises auf die
Patenterteilung:
**20.01.93 Patentblatt 93/03**

(84) Benannte Vertragsstaaten:
**AT BE CH DE FR GB IT LI LU NL SE**

(56) Entgegenhaltungen:
**EP-A- 0 100 752**
**FR-A- 2 261 779**
**FR-A- 2 378 859**
**US-A- 4 014 991**

**COMMONWEALTH AGRICULTURAL BUREAU, Giessen, 14.-15. Juni 1984, Seiten 25-55, Ref.Nr. 84559653 CAB 842249273; L.G. SCHNEIDER: "Feldversuch zur oralen Immunisierung von Füchsen gegen die Tollwut in der Bundesrepublik Deutschland"**

(73) Patentinhaber: **BEHRINGWERKE Aktiengesellschaft**
**Postfach 1140**
**W-3550 Marburg 1(DE)**

(72) Erfinder: **Schneider, Lothar G., Prof. Dr.**
**Brahmsweg 17**
**W-7400 Tübingen(DE)**

(74) Vertreter: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

**Beschreibung**

Die vorliegende Erfindung betrifft einen Street Alabama Dufferin-(SAD)-Varianten-Impfstoff für Tiere gegen Tollwut, der lebendes, attenuiertes Virus enthält. Der Impfstoff kann parenteral oder oral angewendet werden.

Lebendimpfstoffe zur Immunisierung von Tieren sind bereits bekannt. Sie werden üblicherweise parenteral appliziert. Der Nachteil aller Lebendvakzinen ist ihre geringe Haltbarkeit in flüssiger Form. Lebendimpfstoffe werden daher in der Regel lyophilisiert, müssen nach Vorschrift kühl gelagert und nach Rekonstitution umgehend verbraucht werden. Unschädlichkeit und Wirksamkeit dieser Lebendimpfstoffe sind nur nach intramuskulärer Applikation gewährleistet. Nach anderen Applikationsarten, insbesondere subkutaner Injektion, wurden gelegentlich und nach intrazerebraler (i.c.) Injektion regelmäßig Fälle von Impftollwut beobachtet.

Es hat sich gezeigt, daß durch Impfung von Haustieren das Tollwut-Reservoir - vor allem wildlebende Karnivoren - nicht erreicht werden kann. Damit existiert eine permanente Infektionsquelle für nicht-immunisierte Tiere und Menschen.

Die im Can.Vet.Jour. 5, 279-286, 1964 beschriebene ERA-Vakzine wurde an $BHK_{21}$-Zellen adaptiert (Am.J.Epidemiol. 93, 487-490, 1971; $ERA/BHK_{21}$) und erwies sich für Füchse als immunogen und unschädlich, wenn der flüssige Impfstoff auf die Zunge getropft wurde. Das $ERA/BHK_{21}$-Virus war jedoch für Baumwoll- und Bisamratten noch pathogen.

Das Flury HEP-Virus, Klon 675 (Tierärztl. Umschau 37, 165-176, 1982) erwies sich zwar als weitgehend apathogen für eine Reihe von wildlebenden Kleinsäugern, zeigte jedoch eine Neigung zu Latenz, da es noch nach 100 Tagen bei zahlreichen Tieren im Gehirn nachweisbar war. Es ist überdies genetisch nicht stabil und seine Immunogenität für Füchse gering, da auch relativ hohe Virusdosen nicht regelmäßig zum Impferfolg führen.

Die zuverlässigsten Impferfolge bei Füchsen wurden bisher mit dem SAD-Virus, dem auch im ERA-Impfstoff enthaltenen attenuierten Virusstamm in der Schweiz erzielt (Häfliger et al., Zbl.Vet.Med.B, 29, 604-618, 1982).

Ein entscheidender Nachteil des Schweizer SAD-Impfstoffes ist jedoch dessen geringe Stabilität, die zu erheblichen Wirksamkeitsverlusten führen kann. Der Impfstoff kann in Einzeldosen nicht gefroren gelagert werden und muß spätestens am Tage nach der Abfüllaktion im Feld ausgebracht werden. Der Virustiter sinkt infolge von Temperatureinflüssen nach etwa 3 Tagen unter die fuchswirksame Dosis.

Die Erfindung hatte sich daher die Aufgabe gestellt, einen oral applizierbaren Impfstoff zur Bekämpfung der Tollwut in Tieren zu entwickeln, der die aufgezeigten Nachteile nicht hat, der bei oraler Applikation immunogen, unschädlich für Tiere, genetisch stabil, klar durch Marker zu definieren und von Wildstämmen zu unterscheiden und über eine möglichst lange Zeit bei wechselnden Temperaturen stabil ist.

Diese Aufgabe konnte überraschenderweise folgendermaßen gelöst werden: Der Virusstamm $SAD/BHK_{21}$ (Häfliger et al; siehe oben) wurde auf einer Reihe von Zellklonen von BSR-Zellen (Sato, M. et al., Archieves Virology 53, 269-273, 1977) vermehrt und die jeweiligen Virvsausbeuten bestimmt. Der BSR-19 Zellklon (Sato, M. et al.; loc. cit.) wurde durch Klonierung auf Weichagar selektiert. Eine auf diese Weise gewonnene Virusvariante mit einem gleichbleibenden Virustiter von 1-2 $\cdot$ $10^8$ KID50/ml wurde selektiert, als SAD-Variante $VA_1$ bezeichnet und unter Nr. I-430 bei der Collection Nationale de Cultures de Microorganismes (C.N.C.M.), 25 rue du Docteur Roux, 75724 Paris Cedex 15, am 3.4.1985 hinterlegt. Die Selektion erfolgte im Hinblick auf eine hohe Virusausbeute und weitgehende Apathogenität für Mäuse. Das Virus wurde gefriergetrocknet aufbewahrt. Die Zellen des Klons BSR-19 wurden in flüssigem Stickstoff gelagert.

Die Impfvirusvariante $SAD/VA_1$ zeigt im Vergleich mit dem Impfstamm $ERA/BHK_{21}$ eine herabgesetzte Pathogenität sowie eine gegenüber dem Impfstamm $SAD/BHK_{21}$ verbesserte Stabilität und einen besseren Wirkungsgrad.

Von $SAD/VA_1$ ausgehend wurden mittels des monoklonalen Antikörpers MW-187.2.8 durch Passagen in BSR-Zellen die Varianten $SAD/VA_{12}$ (C.N.C.M Nr. I-431)[*] und $SAD/VA_{17}$ (C.N.C.M. Nr. I-432)[*] gewonnen. Von $SAD/VA_{12}$ ausgehend wurde mittels des monoklonalen Antikörpers PM 13 eine weitere Variante mit der Bezeichnung $SAD/VA_{20}$ gewonnen. Das Neutralisationsverhalten der Varianten $VA_{12}$, $VA_{17}$ und $VA_{20}$ gegenüber einer Reihe von bestimmten monoklonalen Antikörpern ist in der Tabelle 1 gezeigt.

[*] Bei der Collection Nationale de Cultures de Microorganismes (C.N.C.M.), 25 rue du Docteur Roux, 75724 Paris Cedex 15, am 3.4.1985 hinterlegt.

Tabelle 1

Monoklonaler Antikörper

| Virus | MW187.2.8 | E529 | C37.12.4 | C231/A | PM13 | PM37 |
|---|---|---|---|---|---|---|
| SAD/Ausgang | 8000[1] | 40000 | 50 | 50 | 0 | 50 |
| SAD/VA$_1$ | 8000 | 300 | 40000 | 1500 | 0 | 25 |
| SAD/VA$_{12}$ | 0 | 0 | 0 | 1500 | 0 | 25 |
| SAD/VA$_{17}$ | 0 | 300 | 0 | 3000 | 0 | 50 |
| SAD/VA$_{20}$ | 0 | 200 | 0 | 0 | 25 | 0 |
| | | | | 300 | | |

1) reziproker Antikörpertiter im Neutralisationstest

Gegenstand der Erfindung ist deshalb ein Lebendimpfstoff zur Immunisierung von Tieren gegen Tollwut, dadurch gekennzeichnet, daß er eine SAD-Virus-Variante enthält, deren Wirksamkeit in flüssiger Form nach mindestens drei Tagen bei mindestens 20°C erhalten bleibt.

Gegenstand der Erfindung ist besonders ein Lebendimpfstoff, dadurch gekennzeichnet, daß er eine der SAD-Varianten VA$_1$, VA$_{12}$, VA$_{17}$ oder VA$_{20}$ enthält.

Gegenstand der Erfindung ist weiterhin ein Verfahren zur Herstellung eines solchen Lebendimpfstoffs, dadurch gekennzeichnet, daß aus dem SAD-Virus-Kollektiv mittels des monoklonalen Antikörpers MW 187.2.8 und gegebenenfalls PM 13 eine der Virus-Varianten SAD/VA$_1$, SAD/VA$_{12}$, SAD/VA$_{17}$ oder SAD/VA$_{20}$ isoliert, eine Suspension einer solchen Variante mit zur Herstellung von Impfstoffen üblichen Hilfsstoffen, Zusatzstoffen und/oder Stabilisatoren versetzt und gegebenenfalls lyophilisiert wird.

Gegenstand der Erfindung ist besonders ein Verfahren zur Herstellung eines solchen Lebendimpfstoffes, dadurch gekennzeichnet, daß eine Suspension einer der Virus-Varianten SAD/VA$_1$, SAD/VA$_{17}$ oder SAD/VA$_{20}$ mit zur Herstellung von Impfstoffen üblichen Hilfsstoffen, Zusatzstoffen und/oder Stabilisatoren versetzt und gegebenenfalls lyophilisiert wird.

Ein solcher Impfstoff kann nach bekannten Methoden unter Verwendung beispielsweise der Zellinie BHK-BSR und einer der in der Anmeldung beschriebenen Varianten VA$_1$, VA$_{12}$, VA$_{17}$ oder VA$_{20}$ hergestellt werden. Die Zellen der Zellinie BHK-BSR können zu diesem Zweck in Einschichtzellkulturen in mehreren Schritten mit einer Splitrate von 1:2 bis 1:15, vorzugsweise 1:3 bis 1:10 vermehrt werden. Die Zellkulturen werden mit einer vorab definierten Viruskonzentration und mit Hilfe der Zellmischtechnik beispielsweise unter Verwendung von 50 g/ml DEAE-Dextran, das durch einen Zentrifugierprozeß aus der Zellkultur wieder entfernt wird, vermehrt. In Abständen von 2-5 Tagen erfolgt die Virusernte durch Abzug des virushaltigen Nährmediums. Die Virusernten werden bei -20°C bis -80°C bis zur Weiterverwendung gelagert. Aus der Virussuspension wird der fertige Impfstoff mit Hilfe eines Puffers, beispielsweise einer 0,1 m-NaCl-Phosphatpufferlösung vom pH 7,2, die Rinderserumalbumin, vorzugsweise 0,3 mol/l, und Eigelb, vorzugsweise 100 g/l, als Stabilisatoren enthält, auf eine Viruskonzentration von vorzugsweise 3-4 x $10^7$ KID$_{50}$ - (Kultur infektiöse Dosen) pro Impfdosis (1,8 ml) eingestellt und vorzugsweise in Durchdrückpackungen, bestehend aus einer PVC-Schüsselfolie und einer thermolackierten Aluminium-Deckblattfolie abgefüllt. Der fertige Impfstoff wird tiefgefroren, beispielsweise bei -20°C bis -35°C, gelagert.

VA$_{12}$, VA$_{17}$ und VA$_{20}$ wurden in Verdünnungen von 1 bis $10^{-5}$ und intrazerebraler, intramuskulärer und oraler Applikation in erwachsenen Mäusen auf Pathogenität geprüft. Keine der 20 infizierten Mäuse in jeder Gruppe erkrankte.

Die Varianten VA$_1$, VA$_{12}$, VA$_{17}$ und VA$_{20}$ wurden durch eine nochmalige Passage in BSR-19-Zellen vermehrt und in bekannter Weise (Häfliger et al., 1982) zu einem oral applizierbaren Impfstoff verarbeitet. Man ließ die Impfstoffe dann in einen Köder verpackt von Füchsen oral aufnehmen.

Wenn die Impfstoffkonzentration $10^5$ oder größer war, betrug, bildeten alle Füchse, die den Impfstoff

3

erhalten hatten, Antikörpertiter im schützenden Bereich, und zwar Titer von 1:65 bis 1:1300.

Alle immunisierten Füchse erwiesen sich zwischen 3 und 13 Monaten nach Applikation als immun. Nebenreaktionen systemischer und lokaler Art wurden nicht beobachtet.

Bei einer Anwendung in der freien Wildbahn wird vorzugsweise eine Antigenkonzentration von $2 \times 10^7$ KID50/ml angewandt. Bei einem entsprechend kontrollierten Feldversuch (Größe etwa 400 $km^2$) hatten von 25 Füchsen, die innerhalb von 6 Monaten nach der Impfung (= Auslegung der Impfstoff enthaltenden Köder) im Impfgebiet erlegt wurden, 20 Füchse (= 80%) Tollwutantikörper mit einem Antikörpertiter von 1:492 durchschnittlich (1:60 bis 1:1620).

Um den tatsächlichen Schutz oder oral immunisierten Füchse gegen eine Infektion mit hochpathogenem Wildtypvirus zu prüfen, wurden 18 Füchse mit dem Impfstamm SAD/VA$_{12}$ in zwei Viruskonzentrationen oral mit Ködern immunisiert und 3 Monate nach der Impfung einer Testinfektion mit pathogenem Wildvirus unterzogen. Parallel hierzu wurden 24 Füchse mit SAD/BHK$_{21}$-Vakzine nach dem Stand der Technik in drei Viruskonzentrationen ebenso immunisiert und testinfiziert. Eine dritte Vakzine französischer Provenienz, die ebenso wie SAD/VA$_{12}$ apathogen für adulte Mäuse war, wurde bei 18 weiteren Füchsen geprüft.

Während alle 18 der mit dem erfindungsgemäßen Impfstoff immunisierten Füchse überlebten, traf dies nur für 42 % der 24 mit SAD/BHK$_{21}$ immunisierten zu. Keines der 18 mit der dritten Vakzine hehandelten Tiere überlebte, ebenso wie 5 nicht geimpfte Füchse.

Weiterhin wurden 13 Füchse mit dem Impfstamm SAD-VA$_1$ ($2 \times 10^7$ IE/ml) oral mit Ködern immunisiert und 1 Jahr nach der Impfung einer Testinfektion mit hochpathogenem Wildtypvirus unterzogen. Alle 13 Füchse überlebten die Testinfektion, während 5 nicht-geimpfte Füchse an Tollwut erkrankten.

Dies zeigt, daß Füchse nach oraler Immunisierung mit SAD-Varianten-Impfstämmenmindestens 1 Jahr lang gegen eine Infektion mit dem Tollwutvirus geschützt sind.

Weiterhin wurden die Varianten VA$_1$ und VA$_{12}$ im Köder an insgesamt 16 mindestens 1-jährige Hunde oral verabreicht (Virusdosis: 2 $\cdot 10^7$ bis 2 $\cdot$ 10$^9$). Alle hatten 3-4 Wochen nach der Impfung Antikörper gegen Tollwut im schützenden Bereich.

Dieser Befund ist überraschend, da es durch orale Applikation herkömmlicher Impfstoffe nicht gelungen ist, Hunde mit ausreichender Sicherheit zu immunisieren.

Es ist neu, daß Hunden durch orale Aufnahme von SAD-Impfvirus-Varianten enthaltenden Ködern ein ausreichender Schutz gegen Tollwut vermittelt werden kann. Damit erschließt sich erstmals die Möglichkeit, in Gebieten, in denen Hunde ähnlich wie bei uns Wildtiere nicht kontrollierbar sind, gegen Tollwut zu immunisieren.

Der Impfstamm SAD/VA$_1$ wurde in unterschiedlichen Konzentrationen oral an erwachsene Mäuse verabreicht, und die Mortalitätsrate im Vergleich zu der von ERA/BHK$_{21}$ bestimmt. Es wurde mit SAD/VA$_1$ eine Restpathogenität von 4,5 % und mit ERA/BHK$_{21}$ eine von 35 % gefunden.

Weiterhin wurde der Impfstamm SAD/VA$_1$ in unterschiedlichen Konzentrationen oral an adulte Bisamratten verabreicht. Keine der 22 inokulierten Bisamratten erkrankte an Tollwut. Im Beobachtungszeitraum bis 106 Tage p.i. war SAD/VA$_1$ weder im infektiösen Zustand noch als Virusantigen nachweisbar. Dagegen war ERA/BHK$_{21}$-Virus in 61 von 95 Bisamratten noch pathogen.

Weiterhin wurde der Impfstamm SAD/VA$_1$ bei 4°C, 22°C und 37°C gelagert und die Virustiter täglich bestimmt. Verglichen mit dem Impfstamm SAD/BHK$_{21}$ des Standes der Technik waren die Inaktivierungsraten des Impfstammes SAD/VA$_1$ bei allen geprüften Temperaturen deutlich geringer, zum Beispiel bei 37°C um nahezu 2 $\log_{10}$-Stufen, das heißt etwa 99%.

Wenn der Impfstamm SAD/VA$_1$ (5 $\cdot$ 10$^7$ IE/ml) bis zu 25 Tagen im Freien gelagert und in ein- bis mehrtägigen Abständen auf Virusgehalt titriert wurde, wurde ein Absinken des Virustiters unter 10$^6$ KID50/ml selbst bei Tagestemperaturen um 40°C (nachts 20°C) nicht beobachtet. Während 14 Tagen im Freien gehaltene Proben wurden an 8 Füchse oral verabreicht. Alle Füchse hatten nach 21 Tagen nachweisbare Antikörper gegen Tollwut im Bereich von 1:250 bis 1:6200.

In Einzeldosen abgefüllter Impfstoff wurde bei -20°C bis zu 6 Monaten gehalten, ohne da ein Titerverlust nachzuweisen war. Der Stamm SAD/BHK$_{21}$ des Standes der Technik kann dagegen nach Abfüllung in Einzeldosen nicht mehr tiefgefroren gelagert werden, sondern muß, um Titerverluste zu vermeiden, unmittelbar im Feld ausgelegt werden.

Beispiel 1

Gewinnung der Varianten VA$_{12}$ und VA$_{17}$

Eine Suspension des Impfstamms SAD/VA$_1$ wurde 1:10 verdünnt, zu gleichen Volumteilen mit einer Lösung des monoklonalen Antikörpers (MAK) M187/2.8 gemischt und 90 min bei 37°C inkubiert. Die Virus/Antikörpermischung wurde anschließend auf etwa 50 Plaqueschälchen verteilt und 5 Tage bei 35°C inkubiert. Entstandene Virusplaques wurden selektiert und nach einer Zwischenvermehrung auf ihr Neutralisationsverhalten gegenüber den selektiven und anderen MAK geprüft. Gleichzeitig wurde die Pathogenität durch i.c., i.m. und orale Applikation an erwachsenen Mäusen untersucht.

Das Neutralisationsverhalten der Varianten $VA_{12}$ und $VA_{17}$ ist in Tabelle 1 gezeigt und die pathogenen Eigenschaften der SAD Varianten $VA_{12}$ und $VA_{17}$ bei erwachsenen Mäusen weiter vorn beschrieben. Selbst nach intrazerebraler Applikation der Varianten wurden keine Erkrankungs- und Todesfälle beobachtet.

Die Varianten $VA_{12}$ und $VA_{17}$ waren jedoch nach i.c. Applikation für Saugmäuse noch pathogen. Wurde Gehirnmaterial der mit der Variante $VA_{12}$ erkrankten Saugmäuse i.c. an erwachsene Mäuse verabreicht, erkrankten diese an Tollwut. Im Gegensatz zur Variante $VA_{12}$ führte die i.c. Verimpfung von Gehirnmaterial von an $VA_{17}$ erkrankten Saugmäusen, selbst nach mehrfachen Saugmauspassagen, nicht zu Tollwuterkrankungen bei erwachsenen Mäusen.

### Beispiel 2

#### Gewinnung der Variante $VA_{20}$

Eine Suspension der mittels eines monoklonalen Antikörpers selektierte SAD Variante $VA_{12}$ (Beispiel 1) wurde zu gleichen Volumteilen mit einer Lösung des monoklonalen Antikörpers PM 37 gemischt und 90 min bei 37°C inkubiert. Die Virus/Antikörpermischung wurde anschließend auf Plaqueschälchen verteilt und 5 Tage bei 35°C inkubiert. Entstandene Virusplaques wurden selektiert und wie in Beispiel 1 beschrieben untersucht.

Das Neutralisationsverhalten der Variante $VA_{20}$ ist in Tabelle 1 gezeigt. Ihre pathogenen Eigenschaften in erwachsenen Mäusen sind weiter vorn beschrieben. Die Variante $VA_{20}$ war nach i.c. Applikation für Saugmäuse noch pathogen. Die i.c. Verimpfung von Gehirnmaterial der an $VA_{20}$ erkrankten Saugmäuse führte, selbst nach mehrfachen Saugmauspassagen, nicht zu Tollwuterkrankungen bei erwachsenen Mäusen.

Der Vorteil von $VA_{20}$ gegenüber $VA_{12}$ und $VA_{17}$ ist die Stabilität der nicht krankmachenden Eigenschaften, die durch Mehrfachselektion mit monoklonalen Antikörpern erzielt wurde.

### Beispiel 3

Die Herstellung des Impfstoffs erfolgt nach den bekannten Regeln, z. B. Requirements der WHO und Forderungen der Europäischen Pharmakopoe. Die Dosierung beträgt in der Regel zwischen 0,5 und 2 ml, kann jedoch auch andere Volumina beinhalten. Wichtig ist hier, daß die wirksame Substanz wie angegeben eingehalten wird.

Zu diesem Zweck wurden 3 Ampullen mit Zellmaterial BSR-12, gelagert bei -180°C, aufgetaut, in einer Mischung aus ME-Medium (ME-Medium = Minimum Essential Medium; Fa. Serva, Heidelberg, BRD; Kat.-Nr. 47294) und 100 ml Kälberserum pro 1 ME-Medium aufgenommen und auf 3 Rouxschalen verteilt. Nach 3 Tagen wurden die BSR-12-Zellen abtrypsiniert, in genügend ME-Medium aufgenommen und auf 15 Rouxschalen verteilt. Nach 4 Tagen wurden die Zellen wiederum abtrypsiniert, in Eagle's-Medium (Glasgow Modifikation) unter Zusatz von 50 g DEAE-Dextran und Virus-SAD $VA_1$, Verdünnung $10^{-3,6}$, aufgenommen, 30 Min. bei + 37°C gerührt, zentrifugiert, in einer Mischung aus 4000 ml ME-Medium und 400 ml Kälberserum aufgenommen und auf 50 Rouxschalen verteilt. Nach 3 weiteren Tagen wurde das Zellwachstumsmedium gegen ein Erhaltungsmedium (Eagle's Medium / Glasgow Modifikation), das 3 ml/l Rinderserumalbumin enthielt, ausgetauscht.

Nach 2 Tagen wurden 4000 ml Antigensuspension, Virustiter $2 \times 10^8$/ml (Titration in BHK-Zellkulturen), und nach weiteren 2 Tagen nochmals 4000 ml, Virustiter $2 \times 10^8$/ml, erhalten.

Diese Suspensionen wurden mit einer 0,1 molaren Natriumchlorid-Phosphatpufferlösung von pH 7,2 die 0,3 ml/l Rinderserumalbumin und 100 g/l Eigelb als Stabilisatoren enthielt auf eine Viruskonzentration von 3-$4 \times 10^7$ $KID_{50}$ (Kultur infektiöse Dosen) pro Impfdosis (1,8 ml) eingestellt und in Durchdrückpackungen, bestehend aus einer PVC-Schüsselfolie und einer thermolackierten Aluminium-Deckblattfolie abgefüllt. Der fertige Impfstoff wurde tiefgefroren bei -20°C bis -35°C gelagert.

## Patentansprüche

1. Lebendimpfstoff zur Immunisierung von Tieren gegen Tollwut, dadurch gekennzeichnet, daß er die Tollwut-Virus-Variante $SAD/VA_1$, $SAD/VA_{12}$ oder $SAD/VA_{17}$, hinterlegt unter den Nummern I-430, I-431 und I-432 bei der Collection Nationale de Cultures de Microorganismes (C.N.C.M.), 25 rue du Docteur Roux, 75724 Paris Cedex 15, enthält.

2. Verfahren zur Herstellung eines Lebendimpfstoffs nach Anspruch 1, dadurch gekennzeichnet, daß eine Suspension einer der Virus-Varianten mit zur Herstellung von Impfstoffen üblichen Hilfsstoffen, Zusatzstoffen und/oder Stabilisatoren versetzt und gegebenenfalls lyophilisiert wird.

**Claims**

1. A live vaccine for the immunization of animals against rabies, which contains the rabies virus variant SAD/VA$_1$, SAD/VA$_{12}$ or SAD/VA$_{17}$, deposited under the numbers I-430, I-431 and I-432 at the Collection Nationale de Cultures de Microorganismes (C.N.C.M.), 25 rue du Docteur Roux, 75724 Paris Cedex 15.

2. A process for the preparation of a live vaccine as claimed in claim 1, which comprises the addition of auxiliaries, additives and/or stabilizers which are customary for the preparation of vaccines to a suspension of one of the virus variants and, where appropriate, freeze-drying.

**Revendications**

1. Vaccin vivant pour l'immunisation d'animaux contre la rage, caractérisé en ce qu'il contient le mutant de virus de la rage SAD VA$_1$, VA$_{12}$ ou VA$_{17}$, déposés sous les numéros I-430, I-431 et I-432 à la Collection Nationale de Cultures de Microorganismes (C.N.C.M.), 25, rue de Docteur Roux, 75724 Paris Cedex 15.

2. Procédé de préparation d'un vaccin vivant selon la revendication 1, caractérisé en ce qu on ajoute à une suspension d'un des virus mutants des adjuvants, des additifs et/ou des stabilisants classiques pour la préparation de vaccins et qu'éventuellement on la lyophilise.